# DEMANDE DE BREVET EUROPEEN

(11) **EP 1 082 950 A1**
(43) Date de publication de la demande: **14.03.2001**
(21) Numéro de dépôt: 00402434.5
(22) Date de dépôt: 05.09.2000
(51) Int. Cl.: A61F 2/44

(54) **Implant intersomatique, dispositif pour la stabilisation du rachis et kit pour la réalisation d'une arthrodèse**

(30) Priorité: 08.09.1999 FR 9911230
(71) Demandeur: PHUSIS, 38330 Saint Ismier (FR)
(72) Inventeur: Benazet, Jean-Pierre, 75014 Paris (FR); Lazennec, Jean-Yves, 94240 L'Hay les Roses (FR); Huet-Olivier, Jacqueline, 38330 Saint-Ismier (FR)
(74) Mandataire: Keib, Gérard

(57) **Abrégé**

L'invention se rapporte à un implant (1), de forme générale parallélépipédique, comprenant deux faces latérales (7) sensiblement parallèles entre elles ; une face antérieure (4) et une face postérieure (5) sensiblement parallèles entre elles ; une face supérieure (2) et une face inférieure (3) sensiblement parallèles entre elles, les faces supérieure (2) et inférieure (3) comprenant des moyens de stabilisation de l'implant (1) entre deux vertèbres consécutives.

L'invention concerne également un dispositif de stabilisation du rachis, comprenant deux implants (1) destinés à être placés de chaque côté de l'apophyse épineuse, ainsi qu'un kit pour la réalisation d'une arthrodèse.

## Description

L'invention se rapporte à un implant intersomatique pour la réalisation d'arthrodèses, notamment lombaires et un dispositif de stabilisation du rachis, notamment lombaire.

L'invention se rapporte également à un kit pour la réalisation d'une arthrodèse, notamment lombaire.

Les pathologies lombaires sont multiples et nécessitent un traitement thérapeutique adéquat.

L'arthrodèse, intervention chirurgicale consistant à bloquer définitivement deux vertèbres consécutives, peut dans certains cas être préconisée.

Différentes techniques d'arthrodèse existent, notamment les greffes intersomatiques par voie antérieure transpéritonéale, les greffes postéro-latérales, les greffes intersomatiques par voie postérieure.

Les autogreffes de tissu cortico-spongieux prélevées par exemple sur la crête iliaque sont couramment utilisées pour la réalisation d'une arthrodèse mais posent des problèmes, notamment de douleur, au niveau du prélèvement.

Une autre technique fait appel à des cages ou implants intersomatiques associés à du greffon, insérés entre deux vertèbres consécutives, pour maintenir un espace intersomatique constant.

Différents implants intersomatiques, disposés de part et d'autre de l'apophyse épineuse en maintenant un écartement distal constant entre les corps vertébraux des vertèbres concernées, ont déjà été proposés.

Ces implants sont généralement agencés pour recevoir un greffon spongieux inséré à l'intérieur de la cage formée par l'implant, et en communication avec les vertèbres à traiter, de façon à permettre une fusion intersomatique.

Par exemple, le document FR-A-2 727 004 décrit un implant de forme générale parallélépipédique, dans lequel est placé un greffon spongieux.

Le document FR-A-2 708 461 décrit un implant de forme générale en tronc de cône, dans lequel sont prévus des orifices permettant l'insertion d'un greffon.

L'implant décrit dans ce document comporte également des nervures externes annulaires dentées, permettant de réduire les mouvements de translation ou de rotation de l'implant, après son insertion entre deux vertèbres.

Les implants disponibles actuellement sont généralement réalisés en un matériau métallique, en carbone ou en titane et présentent des inconvénients.

Par exemple, le contrôle de l'arthrodèse, réalisé à partir d'une image obtenue par imagerie par résonnance magnétique (I.R.M.) ou par scanner peut être délicat à interpréter, en raison de l'apparition d'artéfacts dûs aux matériaux utilisés pour la fabrication de l'implant.

En outre, les implants disponibles actuellement sont délicats à enlever, par exemple en cas de reprise de l'arthrodèse à la suite d'un traumatisme, d'un déplacement de l'implant ou d'une correction à apporter au positionnement de l'implant.

Cette opération peut en effet provoquer des dommages neurologiques et peut remettre en cause la fusion des deux vertèbres.

L'invention a notamment pour but de résoudre les inconvénients ci-dessus, en proposant un implant intersomatique, biorésorbable et radiotransparent.

Un autre but de l'invention est de proposer un implant biorésorbable, dont le maintien au cours du temps est convenable.

A cet effet, l'implant intersomatique selon l'invention, destiné à être placé entre deux vertèbres consécutives, de forme générale parallélépipédique, est réalisé à partir d'un matériau biorésorbable.

L'implant selon l'invention comprend deux faces latérales pleines et sensiblement parallèles entre elles ; une face antérieure et une face postérieure sensiblement parallèles entre elles, la surface externe de la face antérieure étant de dimension plus grande que celle de la surface externe de la face postérieure.

L'implant selon l'invention comprend également deux faces supérieure et inférieure sensiblement parallèles entre elles, comportant chacune au moins une lumière ménagée à leur surface.

Les faces supérieure et inférieure comportent de plus des moyens de stabilisation de l'implant, aptes à assurer le maintien de l'implant entre deux vertèbres consécutives au fur et à mesure de sa résorption.

Selon d'autres caractéristiques, les moyens de stabilisation de l'implant comprennent des éléments en saillie dirigés vers l'extérieur de l'implant, disposés de façon sensiblement régulière sur la surface externe des faces supérieure et inférieure.

Selon une première forme de réalisation, les éléments en saillie ont une forme générale de pyramides, les grandes bases des pyramides étant en contact avec la surface externe des faces supérieure ou inférieure et les sommets des pyramides étant dirigés vers l'extérieur de l'implant.

Selon cette forme de réalisation, les axes longitudinaux médians passant par les sommets des pyramides sont sensiblement perpendiculaires à la surface externe de la face supérieure ou inférieure.

Selon une autre forme de réalisation, les éléments en saillie ont une forme générale d'écaille, comprenant deux parois sensiblement perpendiculaires à la surface externe de la face supérieure ou inférieure, la région inférieure des parois étant en contact avec la surface externe des faces supérieure ou inférieure.

Les deux parois présentent une dimension différente entre elles et sont reliées, au niveau de leur région supérieure, par une face inclinée.

Selon une forme d'exécution, pour une face supérieure ou inférieure donnée, les faces inclinées des écailles d'une région de la face supérieure ou inférieure délimitée par l'intersection des axes longitudinaux et transversaux médians de l'implant sont disposées en opposition avec les faces inclinées des écailles des deux régions adjacentes de la face supérieure ou inférieure considérée.

Il est également envisageable que les écailles prévues sur la face supérieure présentent des faces inclinées disposées en opposition, par rapport à l'axe longitudinal médian de l'implant, avec les faces inclinées des écailles de la face inférieure.

Les mouvements en translation ou en rotation de l'implant, une fois positionnés entre deux vertèbres consécutives, sont ainsi empêchés, et ce au fur et à mesure de la résorption de l'implant.

L'implant selon l'invention comporte également des moyens de fixation d'un préhenseur, pour la mise en place de l'implant selon l'invention.

Les moyens de fixation comprennent un orifice fileté, ménagé sur les faces antérieure et postérieure de l'implant, sensiblement en leur centre, l'orifice étant muni à sa périphérie de deux logements ménagés dans les faces antérieure et postérieure, les axes longitudinaux des logements étant sensiblement parallèles entre eux et passant par le centre de l'orifice.

L'implant selon l'invention est réalisé à partir d'un matériau biorésorbable.

Les matériaux biorésorbables utilisables comprennent les polymères de grande pureté chimique, de masse moléculaire supérieure à environ 250 000, et de faible polydispersité, notamment inférieure à environ 2.

Par exemple, les matériaux biorésorbables comprennent les polymères à base de P-dioxanone, les polyglycolides, ainsi que les dérivés et/ou mélanges de ces produits.

Des matériaux biorésorbables utilisables comprennent également les stéréocopolymères des acides L- et D-lactique, les homopolymères de l'acide L-lactique, les copolymères de l'acide lactique et d'un comonomère compatible, tel que les dérivés d'alpha-hydroxy-acides, ainsi que les dérivés et/ou mélanges de ces produits.

Les matériaux biorésorbables à base d'acide lactique comprennent les polymères contenant au moins 95% de motifs dérivés de l'acide L-lactique.

Les polymères biorésorbables à base d'acide lactique peuvent être préparés selon le procédé décrit dans le document FR-A-2 745 005.

Des exemples de matériaux biorésorbables sont notamment ceux commercialisés sous la marque PHUSILINE.

L'invention vise également à protéger un dispositif de stabilisation du rachis, notamment lombaire et lombo-sacré, comprenant deux implants tels que décrits précédemment destinés à être positionnés de part et d'autre de l'apophyse épineuse.

L'invention vise également à protéger un kit pour la réalisation d'une arthrodèse, notamment lombaire comprenant un dispositif de stabilisation tel que défini précédemment et au moins un préhenseur destiné à la mise en place des implants.

Selon une forme d'exécution, le kit comprend plusieurs dispositifs de stabilisation de dimensions différentes entre eux.

Le kit peut également comprendre des vis pédiculaires, des plaques de liaison, notamment rigides, pour les vis pédiculaires, des écarteurs et fantômes adaptés aux dimensions des implants.

L'invention sera mieux comprise dans la description qui suit, en référence aux figures annexées.

La figure 1 est une vue schématique, en coupe longitudinale, d'un premier mode de réalisation de l'implant. Sur la figure 1, les moyens de stabilisation de l'implant ne sont pas représentés.

La figure 2 représente schématiquement, vu de dessous, l'implant de la figure 1.

La figure 3 représente schématiquement, vu de dessus, l'implant de la figure 1.

La figure 4 est une vue de détail, en coupe longitudinale, de la région A entouré d'un cercle sur la figure 1, représentant de façon schématique les moyens de stabilisation de l'implant.

La figure 5 représente schématiquement, vu de face, l'implant de la figure 1. Sur la figure 5, les moyens de stabilisation ne sont pas représentés.

La figure 6 est une vue schématique, en coupe longitudinale, d'un autre mode de réalisation de l'implant.

Les figures 7 et 8 représentent schématiquement des vues de dessous et de dessus respectivement, de l'implant de la figure 6.

La figure 9 représente schématiquement, vu de face, l'implant de la figure 6. Sur la figure 9, les moyens de stabilisation ne sont pas représentés.

Sur les figures, on désigne par L l'axe longitudinal médian de l'implant et par T l'axe transversal médian de l'implant.

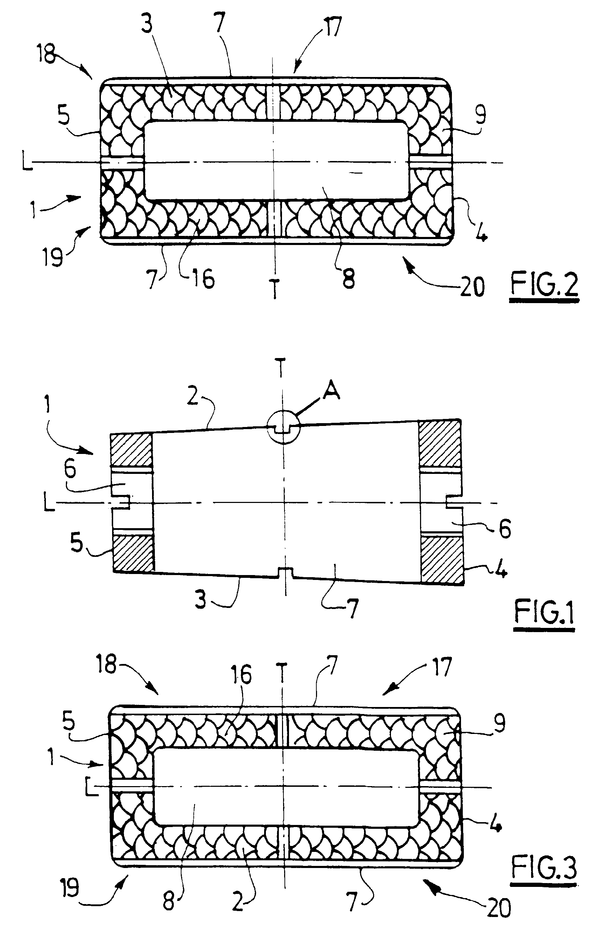

En se référant maintenant à la figure 1, l'implant 1 de l'invention comprend une face supérieure 2 et une face inférieure 3, reliées entre elles par une face antérieure 4 et une face postérieure 5.

La face antérieure 4 présente une dimension plus grande que la face postérieure 5, pour respecter la lordose, notamment lombaire.

Les faces antérieure 4 et postérieure 5 comportent un orifice fileté 6 destiné à la fixation d'un préhenseur utilisé pour la mise en place de l'implant entre deux vertèbres.

L'orifice 6 est muni de deux logements, destinés à coopérer avec des picots ménagés sur le préhenseur pour bloquer toute rotation de l'implant lors de sa mise en place.

Les faces latérales de l'implant sont pleines, de façon à conférer une résistance suffisante à l'implant.

Les faces supérieure 2 et inférieure 3 comportent une lumière 8, ménagée sur une partie de leur surface externe.

La lumière 3 est destinée à l'introduction d'un greffon spongieux, et permet la communication du greffon avec les vertèbres sus- et sous-jacentes pour réaliser la fusion intersomatique.

Il va de soi que les dimensions des lumières 3 ménagées sur les faces supérieure 2 et inférieure 3 de l'implant peuvent varier.

Selon la forme d'exécution représentée sur les figures 2 et 3, les lumières 8 représentent environ la moitié de la surface externe des faces supérieure 2 et inférieure 3.

Il est cependant entendu que la taille des lumières peut être modifiée, notamment en fonction du greffon à introduire, ou du matériau biorésorbable utilisé pour la réalisation de l'implant.

L'implant 1 de l'invention comporte sur ses faces supérieure 2 et inférieure 3 des moyens de stabilisation.

Selon la forme d'exécution représentée sur les figures 2, 3 et 4, les moyens de stabilisation comprennent des éléments en saillie en forme générale d'écailles 9, disposées de façon sensiblement régulière sur la surface externe des faces supérieure 2 et inférieure 3 de l'implant 1.

On décrit ci-après la structure d'une écaille 9, étant entendu que toutes les écailles 9 présentes sur l'implant 1 possèdent une structure identique.

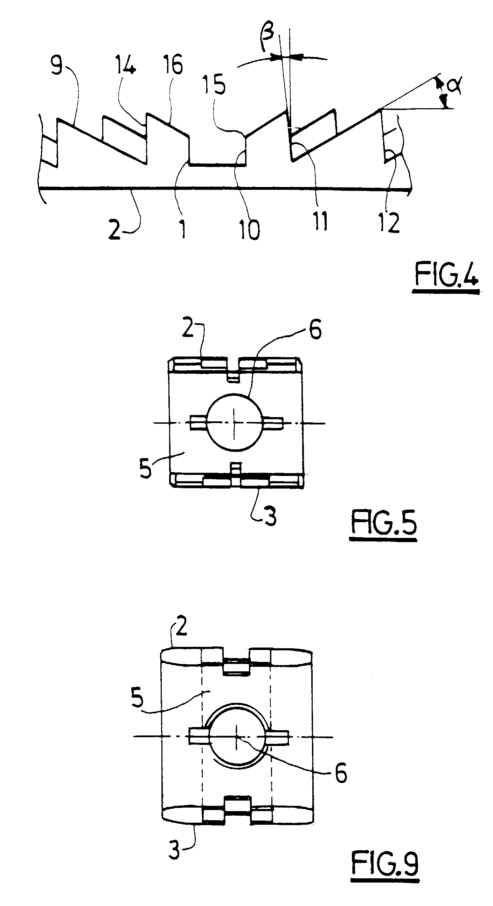

En se référant mainteant plus particulièrement à la figure 4, chaque écaille 9 comprend deux parois 10, 11 sensiblement perpendiculaires à la surface externe de la face supérieure 2.

Les parois 10, 11 comprennent des régions inférieures 12, 13 respectivement, en contact avec la surface externe de la face supérieure 2, les deux parois 10, 11 présentant entre elles une dimension différente.

Les deux parois 10, 11 sont reliées entre elles, au niveau de leurs régions supérieures 14, 15 respectivement, par une face inclinée 16.

La face inclinée 16 est obtenue grâce à la dimension différente des deux parois 10 et 11.

Selon la forme d'exécution représentée, la paroi sensiblement verticale 11 est de contour externe sensiblement circulaire, pour donner aux moyens de stabilisation une forme d'écaille.

La face inférieure 3 de l'implant 1 comporte des moyens de stabilisation d'une structure identique à celle des moyens de stabilisation disposés sur la face supérieure 2 de l'implant.

Les parois 10, 11 des écailles 9 disposées sur la face inférieure 3 sont ainsi sensiblement perpendiculaires à la surface externe de la face inférieure 3 et la région inférieure des parois 10, 11 est en contact avec la surface externe de la face inférieure 3.

Selon la forme d'exécution représentée sur la figure 4, le plan passant par la face inclinée 16 forme avec le plan passant par la surface externe des faces supérieure 2 ou inférieure 3 un angle α d'environ 20° à environ 30°.

Selon cette forme d'exécution, le plan passant par la paroi 11 de plus grande dimension fait avec un plan perpendiculaire à la surface externe de la face supérieure 2 ou inférieure 3 un angle β d'environ 2° à environ 8°.

Selon une forme d'exécution de l'invention, et en se référant plus particulièrement aux figures 2, 3 et 4, les écailles 9 sont orientées différemment sur une même face de l'implant et/ou sur les faces supérieure 2 et inférieure 3 de l'implant 1, pour améliorer encore la stabilisation de l'implant 1 positionné entre deux vertèbres.

Selon ce mode d'exécution, les faces supérieure 2 ou inférieure 3 de l'implant 1 sont divisées chacune en quatre régions, respectivement 17, 18, 19 et 20, délimitées par l'intersection de l'axe longitudinal médian L et de l'axe transversal médian T de l'implant 1.

Pour une face supérieure 2 ou inférieure 3 considérée, les faces inclinées 16 des écailles 9 de chaque région 17, 18, 19, 20 sont orientées en opposition avec les faces inclinées 16 des écailles 9 des deux régions adjacentes de la face supérieure 2 ou inférieure 3 considérée.

Par opposition, il faut entendre dans le cadre de l'invention que les écailles des deux régions adjacentes 17, 18 ou 19, 20 d'une face, situées du même côté de l'axe longitudinal médian L, se font face ou, au contraire, sont tournées dos à dos.

De même, les écailles des deux régions adjacentes 18, 19 ou 17, 20 d'une face, situées de part et d'autre de l'axe longitudinal médian L, sont orientées en sens inverse, par rapport à l'axe transversal médian T de l'implant.

Ainsi, par exemple, les faces inclinées des écailles 9 présentes dans la région 17 de la face supérieure 2 sont en opposition avec les faces inclinées des écailles 9 des deux régions 18 et 20 adjacentes à la région 17 de la face supérieure 2.

De même, les faces inclinées des écailles 9 présentes dans la région 19 de la face inférieure 3 sont en opposition avec les faces inclinées des écailles 9 présentes dans les deux régions 18 et 20 adjacentes à la région 19 de la face inférieure 3.

Les écailles 9 disposées sur la face supérieure 2 peuvent présenter la même orientation que les écailles 9 disposées sur la face inférieure 3.

En variante, et tel que représenté sur les figures 2 et 3, les faces inclinées des écailles 9 présentes sur la face supérieure 2 de l'implant peuvent être orientées, par rapport à l'axe longitudinal médian L de l'implant 1, en opposition avec les faces inclinées des écailles 9 présentes sur la face inférieure 3 de l'implant 1.

Ainsi, par exemple, les faces inclinées des écailles 9 présentes dans la région 19 de la face supérieure 2 sont orientées en opposition avec les faces inclinées des écailles 9 présentes dans la région 19 correspondante de la face inférieure 3 de l'implant 1.

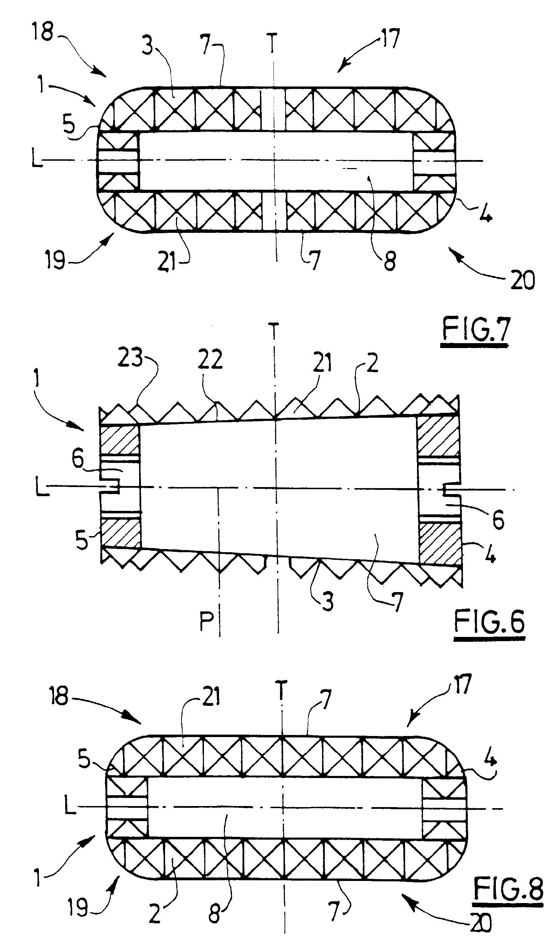

Les figures 6, 7 et 8 illustrent un autre mode de réalisation de l'implant selon l'invention.

Selon ce mode de réalisation, les moyens de stabilisation de l'implant sont différents de ceux utilisés dans le premier mode d'exécution précédent, la structure générale de l'implant étant sensiblement la même que celle décrite dans ce qui précède et n'étant pas reprise ici.

Les moyens de stabilisation comprennent des éléments en saillie en forme générale de pyramides 21, les grandes bases 22 des pyramides 21 étant en contact avec la surface externe des faces supérieure 2 ou inférieure 3 de l'implant 1, et les sommets 23 des pyramides 21 étant dirigés vers l'extérieur de l'implant 1.

L'axe longitudinal médian P passant par les sommets 23 des pyramides 21 est sensiblement perpendiculaire à la surface externe de la face supérieure 2 ou inférieure 3 de l'implant 1.

Les éléments en saillie, qu'il s'agisse des écailles 9 ou des pyramides 21 présentent, par rapport à la surface externe des faces supérieure 2 ou inférieure 3 de l'implant 1, une hauteur variant entre environ 0,5 mm et environ 1,5 mm.

L'implant 1 selon l'invention peut être fabriqué par une technique, classique en elle-même, de moulage par injection d'un matériau biorésorbable tel que défini précédemment, notamment de PHUSILINE.

Sur les figures, des cavités sont représentées sur les faces supérieure et inférieure de l'implant.

Par exemple, en considérant la figure 2, quatre cavités apparaissent sur la face inférieure 3, passant sensiblement par les axes longitudinaux L et transversaux T médians.

Ces cavités résultent de la fabrication par moulage-injection de l'implant et n'ont pas d'utilité en elles-mêmes.

L'implant 1 de l'invention est utilisé pour la réalisation d'arthrodèse, notamment lombaire, par les techniques chirurgicales conventionnelles, par exemple voie postérieure, voie antérieure ou voie postéro-latérale.

L'implant 1 est utilisé dans un dispositif de stabilisation du rachis, notamment lombaire et lombo-sacré, qui comprend deux implants 1 selon l'invention, destinés à être placés de part et d'autre de l'apophyse épineuse.

La mise en place de l'implant est réalisée selon les techniques opératoires actuelles :
- greffe intersomatique par voie antérieure ;
- greffe postéro latérale ;
- greffe intersomatique par voie posétieure.

Lorsque la hauteur intervertébrale souhaitée est obtenue, les deux implants remplis de greffon spongieux sont mis en place alternativement d'un côté de l'apophyse épineuse, puis de l'autre.

Les dimensions différentes des faces antérieure et postérieure de l'implant permettent de conserver ou de restaurer l'espace intersomatique lombaire en conservant ou en redonnant la lordose physiologique de rachis lombaire.

Les éléments en saillie des implants s'engagent dans l'os des vertèbres sus- et sous-jacentes, en interdisant le déplacement axial ou en rotation des implants.

Après la pause, le greffon spongieux, en contact avec l'os des vertèbres sus- et sous-jacentes, assure progressivement la fusion des vertèbres l'une avec l'autre.

La distraction intervertébrale est mise en charge au moyen d'un dispositif complémentaire d'ostéosynthèse, quatre vis pédiculaires reliées transversalement par des plaques, par exemple rigides, ayant été préalablement mises en place dans les vertèbres sus- et sous-jacentes.

## Revendications

1. Implant (1) intersomatique, destiné à être placé entre deux vertèbres consécutives, réalisé à partir d'un matériau biorésorbable, comprenant deux faces latérales (7) pleines et sensiblement parallèles entre elles ; une face antérieure (4) et une face postérieure (5) sensiblement parallèles entre elles, la surface externe de la face antérieure (4) étant de dimension plus grande que celle de la surface externe de la face postérieure (5) ; deux faces supérieure (2) et inférieure (3) sensiblement parallèles entre elles, comportant chacune au moins une lumière (8) ménagée à leur surface, ledit implant étant caractérisé en ce qu'il est de forme générale parallélépipédique et en ce que les faces supérieure (2) et inférieure (3) comporte de plus des moyens de stabilisation de l'implant (1), aptes à assurer le maintien de l'implant (1) entre deux vertèbres consécutives au fur et à mesure de sa résorption.

2. Implant selon la revendication 1, caractérisé en ce que les moyens de stabilisation comprennent des éléments en saillie dirigés vers l'extérieur de l'implant (1), disposés de façon sensiblement régulière sur la surface externe des faces supérieures (2) et inférieure (3).

3. Implant selon la revendication 2, caractérisé en ce que les éléments en saillie ont une forme générale de pyramides (21), les grandes bases (22) des pyramides (21) étant en contact avec la surface externe des faces supérieure (2) ou inférieure (3) et les sommets (23) des pyramides (21) étant dirigés vers l'extérieur de l'implant, les axes longitudinaux médians (P) passant par les sommets (23) des pyramides (21) étant sensiblement perpendiculaires à la surface externe de la face supérieure (2) ou inférieure (3).

4. Implant selon la revendication 2, caractérisé en ce que les éléments en saillie ont une forme générale d'écaille (9), comprenant deux parois (10, 11) sensiblement perpendiculaires à la surface externe de la face supérieure (2) ou inférieure (3), la région inférieure (12, 13) des parois (10, 11) étant en contact avec la surface externe des faces supérieure (2) ou inférieure (3), les deux parois (10, 11) présentant une dimension différente entre elles et étant reliées, au niveau de leur région supérieure (14, 15), par une face inclinée (16).

5. Implant selon la revendication 4, caractérisé en ce que le plan passant par la face inclinée (16) forme avec le plan passant par la surface externe des faces supérieure (2) ou inférieure (3) un angle α d'environ 20° à environ 30°.

6. Implant selon la revendication 4 ou 5, caractérisé en ce que la paroi (11) de plus grande dimension est de contour externe sensiblement circulaire.

7. Implant selon l'une quelconque des revendications 4 à 6, caractérisé en ce que le plan passant par la paroi (11) fait avec un plan perpendiculaire à la surface externe des faces supérieure (2) ou inférieure (3) un angle β d'environ 2° à environ 8°.

8. Implant selon l'une quelconque des revendications 4 à 7, caractérisé en ce que les écailles (9) sont orientées différemment sur une même face (2, 3) de l'implant et/ou sur les faces supérieure (2) ou inférieure (3) de l'implant.

9. Implant selon la revendication 8, caractérisé en ce que, pour une face supérieure (2) ou inférieure (3), les faces inclinées (16) des écailles (9) de chaque région (17, 18, 19, 20) délimitée par l'intersection des axes longitudinaux (L) et transversaux (T) médians sont orientées en opposition avec les faces inclinées (16) des écailles (9) des deux régions adjacentes de la face supérieure (1) ou inférieure (3) considérée.

10. Implant selon la revendication 8 ou 9, caractérisé en ce que les faces inclinées (16) des écailles (9) présentes sur la face supérieure (2) sont orientées en opposition, par rapport à l'axe longitudinal médian (L) de l'implant, avec les faces inclinées (16) des écailles (9) présentes sur la face inférieure (3).

11. Implant selon l'une quelconque des revendications 2 à 10, caractérisé en ce que la hauteur des éléments en saillie, par rapport à la surface externe des faces supérieure (2) ou inférieure (3), est d'environ 0,5 mm à environ 1,5 mm.

12. Implant selon l'une quelconque des revendications 1 à 11, caractérisé en ce qu'il comprend des moyens de fixation d'un préhenseur.

13. Implant selon la revendication 12, caractérisé en ce que les moyens de fixation comprennent un orifice fileté (6), ménagé sur les faces antérieure (4) et postérieure (5), sensiblement en leur centre, l'orifice (6) étant muni à sa périphérie de deux logements ménagés dans la face antérieure (4) et postérieure (5), les axes longitudinaux des logements étant sensiblement parallèles entre eux et passant par le centre de l'orifice (6).

14. Implant selon l'une quelconque des revendications 1 à 13, caractérisé en ce que le matériau biorésorbable comprend les polymères de grande pureté chimique, de masse moléculaire supérieure à environ 250 000, et de faible polydispersité, inférieure à environ 2.

15. Implant selon la revendication 14, caractérisé en ce que le matériau biorésorbable comprend les polymères à base de P-dioxanone, les polyglycolides, ainsi que les dérivés et/ou mélanges de ces produits.

16. Implant selon la revendication 14, caractérisé en ce que le matériau biorésorbable comprend les stéréocopolymères des acides L- et D-lactique, les homopolymères de l'acide L-lactique, les copolymères de l'acide lactique et d'un comonomère compatible tel que les dérivés d'alpha-hydroxy-acides, ainsi que les dérivés et/ou mélanges de ces produits.

17. Implant selon la revendication 16, caractérisé en ce que le matériau biorésorbable comprend les polymères contenant au moins 95% de motifs dérivés de l'acide L-lactique.

18. Dispositif de stabilisation du rachis, notamment lombaire et lombo-sacré, comprenant deux implants selon l'une quelconque des revendications 1 à 17, destinés à être positionnés de part et d'autre de l'apophyse épineuse.

19. Kit pour la réalisation d'une arthrodèse, notamment lombaire, comprenant au moins un dispositif de stabilisation selon la revendication 18 et au moins un préhenseur destiné à la mise en place du dispositif.

20. Kit selon la revendication 19, caractérisé en ce qu'il comprend plusieurs dispositifs de stabilisation de dimensions différentes entre eux.

21. Kit selon la revendication 19 ou 20, caractérisé en ce qu'il comprend de plus des vis pédiculaires et des plaques de liaison, notamment rigides, des vis pédiculaires, des écarteurs et fantômes adaptés aux dimensions des implants.
